(19) Europäisches Patentamt
European Patent Office
Office européen des brevets

(11) Publication number: **0 144 386**
**B1**

(12) # EUROPEAN PATENT SPECIFICATION

(45) Date of publication of patent specification: **06.09.89**

(51) Int. Cl.$^4$: **C 07 H 21/00,** C 07 F 9/65

(21) Application number: **84902180.3**

(22) Date of filing: **14.05.84**

(86) International application number: **PCT/US84/00743**

(87) International publication number: **WO 84/04749 06.12.84 Gazette 84/28**

(54) **NOVEL PREPARATION OF NUCLEOSIDE PHOSPHORAMIDITE INTERMEDIATES.**

(30) Priority: **01.06.83 US 500103**

(43) Date of publication of application: **19.06.85 Bulletin 85/25**

(45) Publication of the grant of the patent: **06.09.89 Bulletin 89/36**

(84) Designated Contracting States: **CH DE GB LI SE**

(56) References cited:
**EP-A-0 061 746**
**EP-A-0 090 789**
**Tetrahedron Letters, volume 24, no. 10, 1983, Pergamon Press Ltd. (GB). Tin M. Cao et al.: "A novel route for solid phase synthesis of polynucleotides using phosphite chemistry", see pages 1019-1020**
**Tetrahedron Letters, volume 24, no. 19, 1983, Pergamon Press Ltd (GB). J.L. Fourrey et al.: "A new and general procedure for the preparation of deoxynucleoside phosphoramidites", pages 1963-1966**

(73) Proprietor: **BECKMAN INSTRUMENTS, INC.**
**Executive Office 2500 Harbor Boulevard Box 3100**
**Fullerton California 92634 (US)**

(72) Inventor: **BEAUCAGE, Serge, Laurent**
**1665 Cornell Drive**
**Mountain View, CA 94040 (US)**

(74) Representative: **Woodin, Anthony John et al**
**Fitzpatricks Europe House Box No. 88 World Trade Centre East Smithfield**
**London E1 9AA (GB)**

(56) References cited:
**Tetrahedron Letters, volume 22, no. 20, 1981, Pergamon Press Ltd (GB). S.L. Beaucage et al.: "Deoxynucleoside phosphoramidites. A new class of key intermediates for deoxypolnucleotide synthesis", pages 1859-1862**
**Tetrahedron Letters, volume 24, no. 3, 1983, Pergamon Press Ltd (GB). L.J. McBride et al.: "An investigation of several deoxynucleoside phosphoramidites useful for synthesizing deoxyoligonucleotides", pages 245-248**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 144 386 B1**

(56) References cited:
Journal of the American Chemical Society, volume 105, no. 3, 9 February 1983 (US). S.P. Adams et al.: "Hindered dialkylamino nucleoside phosphite reagents in the synthesis of two DNA 51-Mers", pages 661-663

Tetrahedron Letters, volume 25, no. 4, 1984, Pergamon Press (GB). S.L. Beaucage: "A simple and efficient preparation of deoxynucleoside phosphoramidites in situ", see pages 375-378

**Description**

## BACKGROUND OF THE INVENTION

1. Field of the Invention

This invention relates to a method for preparing nucleoside phosphoramidite intermediates.

2. Description of the Prior Art

A recent, key innovation in oligonucleotide synthesis was the introduction of the phosphite coupling approach by Letsinger and coworkers (1—3). This approach has been adapted to the synthesis of deoxyoligonucleotides (4—8), oligoribonucleotides (9—12), and nucleic acid analogs (13—15). Generally the approach involves the reaction of a suitably protected nucleoside, a bifunctional phosphitylating agent such as methoxydichlorophosphine, and a second protected nucleoside. Mild oxidation using iodine in tetrahydrofuran, lutidine and water generates the natural internucleotide bond. By varying the oxidation procedure, phosphorus analogs such as selenophosphates (14), imidophosphates (14) and thiophosphates (14, 15) can be generated. A serious limitation of this methodology, however, has been the instability of the reactive intermediates (nucleoside phosphomonochloridites or monotetrazolides) towards hydrolysis and air oxidation. This problem has been circumvented by either preparing the reactive species immediately prior to use or storing the active phosphite as a precipitate in hexanes at −20°C.

The synthesis of a new class of nucleoside phosphites has been proposed in attempt to solve this problem (16). These intermediates have the following formula (16, 17):

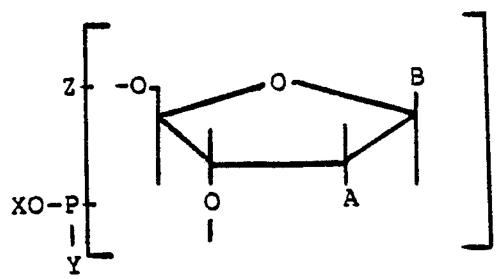

wherein X is a phosphate protecting group; Y is a certain type of secondary amino group; Z is a hydroxy protecting group; A is selected from a group consisting of —H, —OH, and —OZ; and B is selected from a group consisting of purine and pyrimidine bases.

More particularly, Y is a saturated secondary amino group, i.e., one in which no double bond is present in the secondary amino radical. More particulalry, Y is $NR_1R_2$, wherein $R_1$ and $R_2$ taken separately each represents alkyl, aralkyl, cycloalkyl and cycloalkylalkyl containing up to 10 carbon atoms; $R_1$ and $R_2$ when taken together form an alkylene chain containing up to 5 carbon atoms in the principal chain and a total of up to 10 carbon atoms with both terminal valence bonds of said chain being attached to the nitrogen atom to which $R_1$ and $R_2$ are attached; and $R_1$ and $R_2$ when taken together with the nitrogen atom to which they are attached form a saturated nitrogen heterocycle including at least one additional heteroatom from the group consisting of nitrogen, oxygen and sulfur.

Amines from which the group $NR_1R_2$ can be derived include a wide variety of saturated secondary amines such as dimethylamine, diethylamine, diisopropylamine, dibutylamine, methylpropylamine, methylhexylamine, methylcyclopropylamine, ethylcyclohexylamine, methylbenzylamine, methylcyclohexylmethylamine, butylcyclohexylamine, morpholine, thiomorpholine, pyrrolidine, piperidine, 2,6-dimethylpiperidine, piperazine and similar saturated monocyclic nitrogen heterocycles.

The ribonucleoside and deoxynucleoside bases represented by B in the above formulae are well known and include purine derivatives, e.g., adenine, hypoxanthine and guanine, and pyrimidine derivatives, e.g. cytosine, uracil and thymine.

The blocking groups represented by Z in the above formulae include trityl, methoxytrityl, dimethoxytrityl, dialkylphosphite, pivalyl, isobutyloxycarbonyl, t-butyl dimethylsilyl, and similar such blocking groups.

The phosphate protecting groups represented by X include, but are not limited to, a wide variety of hydrocarbyl radicals including alkyl, alkenyl, aryl, aralkyl and cycloalkyl containing up to about 10 carbon atoms. Representative radicals are methyl, butyl, hexyl, phenethyl, benzyl, cyclohexyl, phenyl, naphthyl, allyl and cyclobutyl. Of these the preferred are lower alkyl, especially methyl and ethyl.

These compounds were reported to be not as reactive and not as unstable as prior art compounds. It was noted that these compounds do react readily with unblocked 3′-OH or 5′-OH of nucleosides under normal conditions. In addition, these phosphoramidites were sasid to be stable under normal laboratory conditions to hydrolysis and air oxidation, and to be storable as dry, stable powders.

Recently, the stability of these nucleoside phosphoramidites in acetonitrile solutions has been questioned (18, 19). Additionally, it has been pointed out that access to these nucleoside phosphoramidites is difficult because their isolation is uneasy and time consuming (20, 21). Furthermore, their prohibitive expense contributes to restricting their use to mainly skilled personnel.

In order to alleviate these drawbacks, a few approaches to deoxynucleotide (DNA) solid-state synthesis using phosphite chemistry have been proposed (20, 21). The key features of these procedures involve the phosphitylation of the solid support with bifunctional phosphitylating agents such as methoxydichlorophosphine or bis-(tetrazolyl)-methoxyphosphine. In the latter case the deblocked and supported nucleoside is activated with an excess of the bifunctional reagent to yield an activated nucleosidophosphoromonotetrazolide resin with exposed phosphorous moiety to which a further nucleoside may be bound. Subsequently the phosphite triester is conventionally oxidised and the cycle may be repeated to obtain oligo/polynucleotides. A further prior proposal (47) involves the successive reaction of a 3'-OH free deoxynucleoside with an alkyloxy (or aryloxy) *bis*-triazolylphosphine and a trimethylsilylated secondary amine to yield desirable quantities of phosphoramidite instead of the undesired phosphite. However, these approaches to DNA synthesis appear prone to reproducibility problems because a minute amount of moisture could hydrolyze the very reactive phosphorous moiety immobilized on the solid support either prior to or during the chain elongation step.

Other problems include a problematic aqueous work-up required to remove hygroscopic amine hydrochlorides and destroy unwanted phosphitylations of the heterocyclic rings generated during the preparation of deoxynucleoside phosphoramidites (16); a difficult isolation procedure consisting of the precipitation of toluene or ethyl acetate solutions of the above deoxynucleoside phosphoramidites in cold hexanes (−70°C) (16); stability problems created by extended storage of these deoxynucleoside phosphoramidites in acetonitrile solution; and the use of pyrophoric and/or reactive phosphitylating agents such as chloro-(N,N-dimethylamino)methoxyphosphine (16), chloro-(N,N-diisopropylamino)methoxyphosphine (18), methoxydichlorophosphine (20, 21), bis-(tetrazolyl)methoxyphosphine (21) which are difficult to handle and extremely sensitive to atmosphere moisture.

Accordingly, there exists a need for a simplified methodology.

## SUMMARY OF THE INVENTION

In accordance with the present invention, there is provided a simplified methodology which is readily accessible to automation and, if a manual solid-phase deoxynucleotide (DNA) and/or ribonucleotide (RNA) synthesis is preferred, to use by a non-chemist. More particularly, the present invention encompasses a convenient method for preparing deoxynucleoside and ribonucleoside phosphoramidite intermediates (IV) *in situ* from corresponding natural and unnatural nucleosides. The nucleoside phosphoramidite intermediates prepared in this manner can then be activated with a suitable agent and the activated nucleoside phosphoramidite intermediates can be used in excess during a coupling reaction with a suitably protected nucleoside, thus ensuring a favorable competition against trace amounts of moisture from both the solvent and the surrounding environment.

In one embodiment of the present invention, the nucleoside phosphoramidite intermediate (IV) is prepared by a chemical reaction represented by a formula:

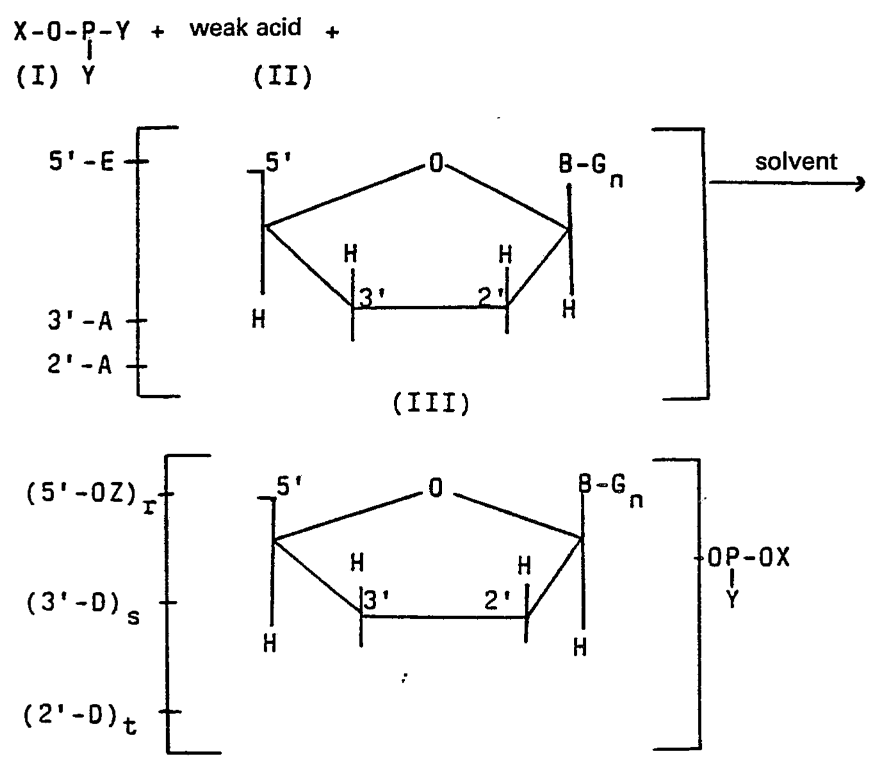


(I) (II) (III)

(IV)

In another embodiment of the present invention, the nucleoside phosphoramidite intermediate (IV) is prepared by a chemical reaction represented by the following formula:

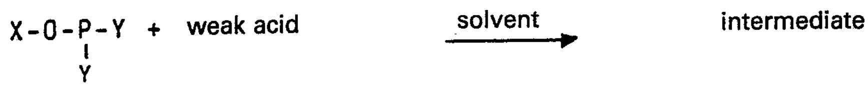


(I) (II)

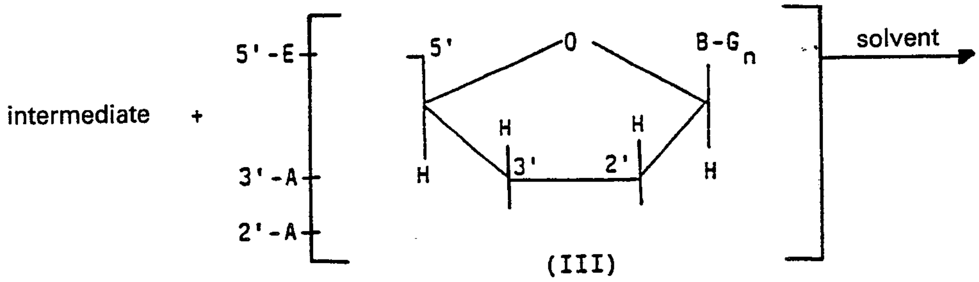


(III)

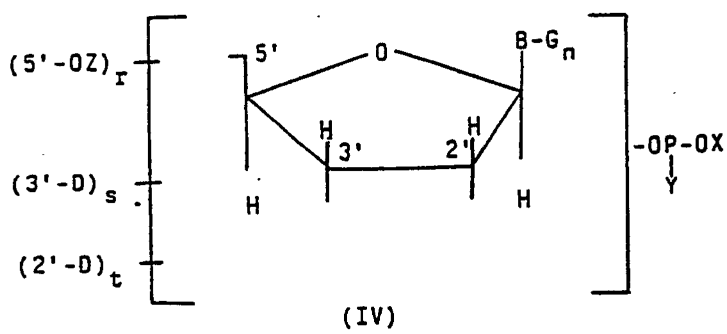

(IV)

In both of the above chemical reaction formulas, X is a phosphate protecting group; each Y is a secondary amino group; Z is a hydroxy protecting group; the weak acid is capable of selectively protonating only one amine of the secondary amino groups; each A is selected from a group consisting of —H, —OH and —OZ; B is selected from a group consisting of natural and unnatural purine and pyrimidine bases; G is a heterocyclic base protecting group; each D is selected from a group consisting of —H and —OZ; E is selected from a group consisting of —OH and —OZ, provided that (a) when both As are the same and are —H and —OZ then E is —OH and (b) when one A is —OH then E is —OZ; n is a number from 0—2; r, s and t are ech 0 or 1 provided that $r + s + t = 2$; the solvent is capable of (a) solubilising I, II and III and (b) allowing the reaction to proceed; and III is present in a molar concentration at least equal to the molar concentration of reactable I (i.e. some of I can be destroyed by moisture or other contaminants in the reaction medium and the portion so lost is not included in this ratio).

Also within the scope of the invention is a novel compound having a formula

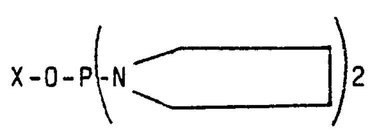

wherein X is methyl.

## DESCRIPTION OF THE PREFERRED EMBODIMENTS

X can be virtually any phosphate protecting group. Many phosphate protecting groups are known to those skilled in the art (31—39). Preferably, X is selected from alkyl, haloalkyl, cyanoalkyl, alkyldiarylsilylalkyl, trialkylsilylalkyl, arylsulphonylalkyl, alkylthioalkyl, arylthioalkyl, alkenyl, haloalkenyl, aryl, haloaryl, aralkyl, haloaralkyl, cycloalkyl, halocycloalkyl, branched alkyl and branched haloalkyl containing up to 10 carbon atoms. More preferably, X is methyl.

Each Y is preferably $R_1$—N—$R_2$, wherein each $R_1$ and $R_2$, when taken separately, represents alkyl, haloalkyl, alkenyl, aralkyl, haloaralkyl, cycloalkyl, halocycloalkyl, cycloalkylalkyl, halocycloalkylalkyl, cycloalkenylyl, halocycloalkenylyl containing up to 10 carbon atoms, wherein each halogen substitution is at least three carbon atoms removed from the nitrogen atom and each unsaturation is at least 2 carbon atoms removed from the nitrogen atom (i.e., the halogen substitution and/or unsaturation should not significantly decrease the basicity of the amino functionality); $R_1$ and $R_2$, when taken together, form a chain

selected from a group consisting of alkylene, haloalkylene, alkenylene, and haloalkenylene chains containing up to 5 carbon atoms in the principal chain and a total of up to 10 carbon atoms with both terminal valence bonds of said chain being attached to the nitrogen atom to which $R_1$ and $R_2$ are attached and wherein each halogen substitution atom is at least three carbon atoms removed from the nitrogen atom and each unsaturation is at least 2 carbon atoms removed from the nitrogen atom; $R_1$ and $R_2$, when taken together with the nitrogen atom to which they are attached, form a saturated nitrogen heterocycle including at least one additional heteroatom selected from a group consisting of N, O, and S. More preferably, each Y is selected from a group consisting of dimethylamine, diethylamine, diisopropylamine, dibutylamine methylpropylamine, methylhexylamine, methylcyclopropylamine, ethylcyclohexylamine, methylbenzylamine, methylcyclohexylmethylamine, butylcyclohexylamine, morpholine, thiomorpholine, pyrrolidine, piperidine, and 2,6-dimethylpiperidine. Optimally, Y is pyrrolidine.

Z can be virtually any hydroxy protecting group. Many hydroxy protecting groups are known to those skilled in the art (31—33, 38—46). Preferably, Z is selected from a group consisting of pixyl groups, (e.g., 9-arylxanthen-9-yl), triarylmethyl groups (e.g., trityl, methoxytrityl, trimethoxytrityl, di-p-anisylphenylmethyl, p-fluorophenyl-1-naphthylphenylmethyl, p-anisyl-1-naphthylphenylmethyl, di-o-anisyl-1-naphthylmethyl, di-o-anisylphenylmethyl, and p-tolyldiphenylmethyl), dialkyl phosphite groups, alkylcarbonyl groups (e.g., acetyl, pivaloyl), arylcarbonyl groups (e.g., benzoyl), trialkylsilylalkyloxycarbonyl groups, aryloxycarbonyl groups, alkoxycarbonyl groups (e.g., isobutyloxycarbonyl), arylsulfonylalkoxycarbonyl groups, arylthioalkyloxycarbonyl groups, aralkyl groups, alkyl groups, alkenyl groups trialkylsilyl groups, ketal functions (e.g., tetrahydropyranyl, 4-methoxytetrahydropyran-4-yl), and acetal functions.

Various nucleosides capable of being employed in the instant invention include nucleosides 7, 3, 5—9 and 12—15 set forth in Table I.

TABLE I

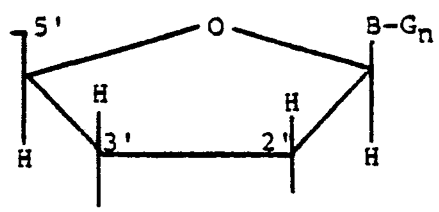

| Nucleoside | 2'-A | 3'-A | 5'-E |
|---|---|---|---|
| 1 | -H | -H | -OH |
| 2 | -H | -OH | -OH |
| 3 | -H | -OH | -OZ |
| 4 | -OH | -H | -OH |
| 5 | -OH | -H | -OZ |
| 6 | -H | -OZ | -OH |
| 7 | -H | -OZ | -OZ |
| 8 | -OZ | -H | -OH |
| 9 | -OZ | -H | -OZ |
| 10 | -OH | -OH | -OZ |
| 11 | -OH | -OZ | -OH |
| 12 | -OH | -OZ | -OZ |
| 13 | -OZ | -OH | -OH |
| 14 | -OZ | -OH | -OZ |
| 15 | -OZ | -OZ | -OH |

Most preferably, the nucleoside has the formula represented by nucleosides 3, 5, 12 and 14 of Table I.

When the nucleoside contains two —OZ substituents, it is essential that one Z protecting groups be capable of deprotection without deprotecting the other. This can be accomplished by any technique known to those skilled in the art. For example, if one Z is trityl and the second Z is selected from a group consisting of benzoyl, trialkylsilyl, tetrahydropyranyl, or other ketal or acetal functions, the trityl can be removed by a mild acid or Lewis acid (e.g., zinc bromide) without removing the other protecting group. Similarly, if one

protecting group is trityl and the other is benzoyl, the benzoyl group can be removed under basic conditions without removing the trityl group. In like fashion, if one protecting group is trityl and the other is trialkylsilyl, the trialkylsilyl group can be removed by treatment with fluoride ions without removing the trityl group.

B can be virtually any natural or unnatural ribonucleoside or deoxynucleoside base. Such bases include, but are not limited to, purine derivatives, e.g., adenine, hypoxanthine and guanine, pyrimidine derivatives, e.g., cytosine, uracil, and thymine, as well as homologues and analogues thereof.

G can be virtually any heterocyclic base protecting group. Many heterocyclic base protecting groups are known to those skilled in the art (23—33). Preferably, G is selected from a group consisting of amino, imide, and amide protecting groups of the corresponding heterocyclic base. Such amino, imide, and amide protecting groups include, but are not limited to triarylmethyl, trialkylsilylalkyl, arylthioalkyl, arylalkyl, cyanoalkyl, phthaloyl, aryl, aryloxycarbonyl, alkoxycarbonyl, arylcarbonyl, alkylcarbonyl, and N-dialkylaminomethylene.

The number n is selected such that one or more of the reactive functionalities on the heterocyclic ring are protected. An illustration of this point is set forth in Table II.

TABLE II

| Heterocyclic Ring | Maximum Reactive Functionalities | Range of n | Preferred Range of n |
|---|---|---|---|
| thymine | 1 | 0-1 | 0 |
| cystosine | 1 | 0-1 | 1 |
| adenine | 1 | 0-1 | 1 |
| guanine | 2 | 0-2 | 1-2 |
| hypoxanthine | 1 | 0-1 | 0 |
| uracil | 1 | 0-1 | 1 |

Preferably, n is 1.

Preferably, the weak acid has a pK at about 25° C in $H_2O$ of about 7.5 to about 11, more preferably from about 8 to about 10.5, even more preferably from about 8 to about 9, and optimally about 8.2 to about 8.5. In addition, the weak acid is preferably selected from a group consisting of 1,2,4-triazole; 1,2,3-triazole; 4,5-dicloroimidazole), 4-nitroimidazole; 3-chlorotriazole; benzotriazole; and mixtures thereof. More preferably, the weak acid is selected from said group consisting of 4,5-dichloroimidazole, benzotriazole, and mixtures thereof. Optimally, the weak acid is 4,5-dichloroimidazole.

Preferably, the solvent is selected from a group consisting of tetramethyl urea, N,N-dimethylacetamide, 1-methyl-2-pyrrolidinone, N,N-dimethylformamide (DMF), dioxane, tetrahydrofuran (THF), ethyl acetate, chloroform, 1,3-dimethyl-2-imidazolidinone, N,N'-dimethyl-N,N'-propyleneurea, homologues and analogues thereof, and mixtures thereof. More preferably, the solvent is selected from a group consisting of tetramethyl urea, 1,3-dimethyl-2-imidazolidinone, 1-methyl-2-pyrrolidinone, and mixtures thereof. In addition, it is also preferred that the solvent be anhydrous (i.e., contain less than 0.06% water).

The chemical reaction can proceed at any convenient temperature. This temperature can range from the freezing point to the boiling point of the reaction mixture. Preferably, the chemical reaction takes place at about room temperature.

Any convenient time can be employed in conducting the chemical reaction. For example, the reaction time can be from a few seconds to over a day. However, it is preferred to conclude the reaction as soon as possible (in a tme span typically from about 5 to about 15 minutes, and more preferably about 10 minutes).

Preferably, III is present in a molar concentration greater than reactable I. More preferably, the molar ratio of III:reactable I is 1.2:1.

The relative molar concentration of I and II is not critical. However, it is preferred that the molar concentration of II be at least twice the molar concentration of I. More preferably the molar concentration of II is from about 2 to about 5 times the molar concentration of I. Optimally, the molar concentration of II is approximately quadruple that of I.

The reactants can be added in any convenient sequence. However, with respect to the one-step rotation embodiment of the present invention it is preferred to add I to a solution containing III. This preferred sequence appears to generate less side products during the course of the reaction.

The nucleoside can be phosphorylated at either the 2′, 3′ or 5′ position and is preferably phosphorylated at the 3′ or 5′ position. More preferably, the nucleoside is phosphorylated at the 3′ position.

The thus formed nucleoside phosphoramidite intermediates can be activated with a suitable agent, for example, 1H-tetrazole and an excess of the activated nucleoside phosphoramidite intermediate can be used during a coupling reaction with a suitably protected nucleoside in either any liquid or solid phase methodology known to those skilled in the art (16, 22). This procedure thus ensures a favorable competition against trace amounts of moisture from the solvent as well as from the surrounding environment.

It is preferred that the coupling reaction be performed in a solid phase procedure.

The following examples are provided for the purpose of further illustration only and are not intended to be limitations on the disclosed invention.

## Example 1

Preparation of bis-(N,N-dimethylamino)methoxyphosphine

To 0.1 mole of methoxydichlorophosphine cooled at −15°C in a 100 ml round bottom flask, was added under a nitrogen atmosphere dropwise over a period of about 30 minutes N,N-dimethylaminotrimethylsilane (0.21 mole). The reaction mixture was then removed from the cold bath and allowed to stir at ambient temperature for about 1 hour. The chlorotrimethylsilane generated during the course of the reaction was removed under reduced pressure (water aspirator). The material left distilled at 38—40°C at 13 mm Hg. The cloudy liquid was filtered through a medium porosity glass sintered funnel. The yield was 68%. Physical characteristics were identified as follows:

$^1$H NMR ($CDCl_3$):

3.5 ppm (d, —$OCH_3$, 3H) $J_{P-H}$ = 13 Hz

2.7 ppm (d, —$N(CH_3)_2$, 12H) $J_{P-H}$ = 9 Hz

The spectrum was recorded wth respect to external TMS reference.

$^{31}$P NMR ($CDCl_3$: −138.18 ppm with respect to external 85% $H_3PO_4$ reference.

The density of the clear liquid at 25°C was found to be 0.936 g/ml.

## Example 2

Preparation of bis(pyrrolidino)methoxyphosphine

To 0.1 mole of methoxydichlorophosphine cooled at −15°C in a 100 ml round bottom flask, was added under a nitrogen atmosphere, dropwise over a period of about 30 minutes N-trimethylsilylpyrrolidine (0.21 mole). The reaction mixture was then removed from the cold bath and allowed to stir at ambient temperature for about 1 hour. The chlorotrimethylsilane generated during the course of the reaction was removed under reduced pressure (water aspirator). The material left distilled at 66—68°C at 0.16 mm Hg. The cloudy liquid was filtered through a medium porosity glass sintered funnel. The yield was 82%. Physical characteristics were identified as follows:

$^1$H NMR ($CDCl_3$):

3.4 ppm (d, —$OCH_3$) $J_{P-H}$ = 13 Hz

3.1 ppm (m, —$NCH_2$)

1.8 ppm (m, —$CH_2$—$CH_2$—)

The spectrum was recorded with respect to external TMS reference.

$^{31}$P NMR ($CDCl_3$: −134.01 ppm with respect to 85% $H_3PO_4$ reference.

The density of the clear liquid at 25°C was found to be 1.037 g/ml.

## Example 3

Preparation of bis-(N,N-diisopropyl)methoxyphosphine

To methoxydichlorophosphine (15 g, 0.11 mole) in 100 ml of anhydrous ethyl ether was added, dropwise at −15°C under a nitrogen atmosphere over a period of about 30 minutes, N,N-diisopropylamine (44.5 g, 0.44 mole) in 100 ml of anhydrous ethyl ether. The suspension was then removed from the cold bath and allowed to stir at ambient temperature for about 1 hour. The amine hydrochloride was then filtered and washed with 100 ml of anhydrous ether. Excess ether from the filtrate was distilled off at atmospheric pressure. The material left was freed from residual amine hydrochloride by filtration and was then distilled under high vacuum to afford a fraction boiling at 59—61°C at 0.2 mm Hg. The yield was 79%. Physical characteristics were identified as follows:

$^1$H NMR ($CDCl_3$):

3.3 ppm (d, —$OCH_3$) $J_{P-H}$ = 14 Hz

3.4 ppm (m, —$N(CH)_2$)

1.0 ppm (d, —$Ch(CH_3)_2$

$^{31}$P NMR ($CDCl_3$: −131.8 ppm with respect to external 85% $H_3PO_4$ reference.

The density of the clear liquid at 26°C was found to be 0.911 g/ml.

Example 4

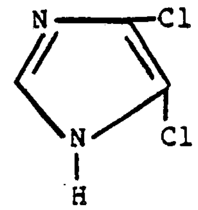

+

II

$CH_3O-P-(Y)_2$ + 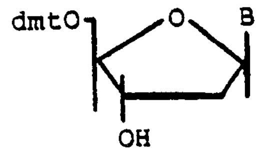 solvent

I a, Y = 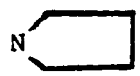

III, B = 1-Thyminyl

dmt = di-p-anisylphenylmethyl

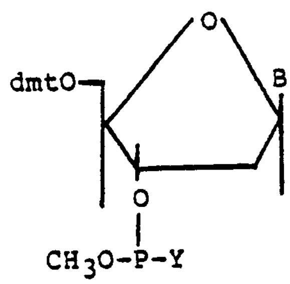 + 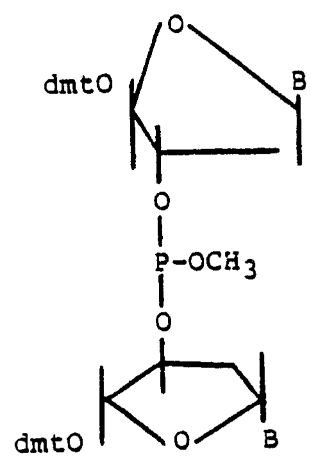

IV a

V

The bis-(pyrrolidino)methoxyphosphine (Ia; 0.25 mmol) prepared in Example 2 was syringed into a solution of the 5'-protected deoxynucleoside (III; 0.28 mmol) and 4,5-dichloroimidazole (II; 1.00 mmol) in 0.5 ml of dry 1-methyl-2-pyrrolidinone, $^{31}P$ NMR displayed a nearly quantitative yield of the nucleoside phosphorimidite IVa (−143.7, −143.4 ppm; 91.2% yield) along with a small amount of the symmetrical dinucleoside monophosphite V (−139.7 ppm; 8.8% byproduct).

Example 5

Oligomer Preparation

In order to test the new synthetic methodology toward polynucleotides synthesis, the following experiment was undertaken. To a solution of the 5'-protected nucleoside Ia (153 mg, 0.28 mmol) and 4,5-dichloroimidazole (II, 136 mg, 1.00 mmol) in dry 1-methyl-2-pyrrolidinone (0.5 ml) was added under a nitrogen atmosphere at ambient temperature, 50 µl of bis-(pyrrolidino) methoxyphosphine, (Ia; 0.25 mmol). After about 10 minutes, 70 µl (25 mmol) of the stock solution was syringed into a 4 ml vial containing 30 mg of solid support derivatized with 1 µmole of deoxythymidine suspended in 250 µl of a 0.5M acetonitrile solution of 1H-tetrazole. The reaction mixture was shaken for about 5 minutes at ambient temperature, quenched with 1.5 ml of a solution of THF:2,6-Lutidine:$H_2O$ (2:1:2) and oxidized according to the usual procedure. After the standard deprotection, the reaction products were analyzed by high performance liquid chromatography (HPLC). A high yield of d(TpT) (>95%) and some unreacted deoxythymidine (<5%) were observed. d(CpT), d(ApT) and d(GpT) were also produced in yields exceeding 95%. These products were rigorously identical to authentic samples commercially available.

Example 6

According to the procedure set forth in Example 5 the following oligonucleotide has been successfully prepared:

d(GCATCGCCAGTCACTATGGCGT)

The overall yield was about 30%, implying that an average yield of 94.4% was obtained at each step (as measured spectrometrically from the dimethoxytrityl cation using an extinction of 7 × $10^4$ at 498 nm).

The synthetic approach disclosed herein, using a selective activation of compounds of formula I for the preparation of nucleoside phosphoramidites *in situ* is an improved route for automated solid-phase DNA and RNA synthesis. Some of the advantages of this methodology over the existing ones are as follows:

The elimination of the aqueous work-up required to remove hygroscopic amine hydrochlorides and destroy unwanted phosphitylations of the heterocyclic rings generated during preparation of deoxynucleoside phosphoramidites. (16).

Elimination of difficult isolation procedure consisting in the precipitation of toluene or ethyl acetate solutions of the deoxynucleoside phosphoramidites in hexanes (−70°C) (16).

Elimination of stability problems created by extended standing of the deoxynucleoside phosphoramidites in acetonitrile solutions because the deoxynucleoside phosphoramidite intermediates made *in situ* will generally be consumed within a relatively short period of time.

The elimination of pyrrophoric and/or reactive phosphitylating agents such as chloro-(N,N-dimethylamino)methoxyphosphine (16), chloro(N,N-diisopropylamino)methoxyphosphine (18), methoxydichlorophosphine (20, 21) bis-(tetrazolyl)methoxyphosphine (21), which are difficult to handle and extremely sensitive to atmospheric moisture.

Compounds of formula I are easily prepared and safe to handle, because they are extremely stable toward atmospheric moisture relative to the corresponding chlorophosphines.

Accordingly, the use of stable protected nucleosides which are transformed into nucleoside phosphoramidite intermediates in high yield via a selective activation of compounds of formula I, combined with a high coupling yield through efficient activation of the *in situ* generated nucleoside phosphoramidite intermediates by an appropriate activating agent makes the methology of the present invention accessible to automation and, if a manual solid-phase or liquid phase DNA and/or RNA synthesis is preferred, to the non-chemist.

REFERENCES

1. Letsinger et al., *J. Am. Chem. Soc., 97,* 3278—3279 (1975).
2. Letsinger et al., *J. Am. Chem. Soc., 98,* 3655—3661 (1976).
3. Letsinger et al., *Proc. Int. Conf. Transfer RNA,* Poznan, Poland, October 1976, pp. 145—155.
4. Matteucci et al., *Tetrahedron Lett., 21,* 719—722 (1980).
5. Finnan et al., *Nucl. Acids Res. Symposium series No. 7,* 133—145 (1980).
6. Caruthers, *Proc. of IUPAC International Symposium on Macromolecules,* Florence, Italy, September 1980, Pergamon Press, Oxford, England.
7. Caruthers et al., *Nucl. Acids Res. Symposium Series No. 7,* 215, 223 (1980).
8. Matteucci et al., *J. Am. Chem. Soc., 103,* 3185—3191 (1981).
9. Daub et al., *J. Am. Chem. Soc. 99,* 3526—3528 (1977).
10. Ogilvie et al., *Can. J. Chem. 58,* 1389—1397 (1980) and references therein. See also Ogilvie et al., *Can. J. Chem., 58,* 2686—2693 (1980).
11. Ogilvie et al., *Nucl. Acids Res. Symposium Series No. 7,* 147—150 (1980).
12. Ogilvie et al., *Tetrahedron Lett., 21,* 4159—4162 (1980).
13. Melnick et al., *J. Org. Chem., 45,* 2715—2716 (1980).
14. Ogilvie, *Tetrahedron Lett., 21,* 4145—4848 (1980); *ibid.,* 4149—4152 (1980; 4153—4154 (1980).
15. Burgers et al., *Tetrahedron Lett.,* 3835—3838 (1978).
16. Beaucage et al., *Tetrahedron Lett., 22,* 1859—1862 (1981).
17. Unpublished U.S. Patent Application.
18. McBride et al., *Tetrahedron Lett., 24,* 245—2486 (1983).
19. Adams et al., *J. Am. Chem. Soc., 105,* 661—663 (1983).
20. Jayaraman et al., *Tetrahedron Lett., 23,* 5377—5380 (1982).
21. Cao et al., *Tetrahedron Lett., 24,* 1019—1020 (1983).
22. Caruthers et al., *Genetic Engineering,* eds. Setlow et al., Phenum Press, N.Y. (1982) pp. 1—17.
23. Sekine et al., *J. Org. Chem., 47,* 571—573 (1982).,
24. Kume et al., *Tetrahedron Lett., 23,* 4365—4368 (1982).
25. Trichtinger et al., *Tetrahedron Lett., 24,* 711—714 (1983).
26. Gaffney et al., *Tetrahedron Lett., 23,* 2257—2260 (1982).
27. Hata et al., *Bull. Chem. Soc. Japan, 55,* 2949—2955 (1982).
28. Reese et al., *Tetrahedron Lett., 22,* 4755—4758 (1981).
29. Rappoport et al., *J. Am. Chem. Soc., 104,* 5702—5708 (1982).
30. Sekine et al., *Tetrahedron Lett., 23,* 5287—5290 (1982).
31. Reese, *Tetrahedron, 34,* 3143—3179 (1978).

32. Othsuka et al., *Nucl. Acids Res.*, *10*, 6553—6570 (1982).
33. Amarnath et al., *Chem. Rev.*, *77*, 183—217 (1977).
34. Josephson et al., *Chem. Scripta*, *18*, 184—188 (1981).
35. Charubala et al., *Tetrahedron Lett.*, *23*, 4789—4792 (1982).
36. Reese et al., *Tetrahedron Lett.*, *24*, 951—954 (1983).
37. Kraszwoski et al., *Nucl. Acids Res. Symposium Series No. 9*, 135—139 (1981).
38. Ikehara et al., *Adv. Carb. Chem. Biochem.*, *36*, 135—213 (1979).
39. Kossel et al., *Fortschr. Chem. Org. Natur.*, *32*, 297—508 (1975).
40. Reese et al., *J.C.S. Chem. Comm.*, 987—988 (1979).
41. Hayakawa et al., *Tetrahedron Lett.*, *24*, 1165—1168 (1983).
42. Takaku et al., *Chem. Lett.*, 189—192 (1982).
43. Chattopadhyaya et al., *J.C.S. Chem. Comm.*, 639 (1978).
44. Balgobin et al., *Tetrahedron Lett.*, 3667 (1981).
45. Josephson et al., *Tetrahedron Lett.*, 4537 (1981).
46. Fisher et al., *Nucl. Acids Res.*, *11*, 1589—1599 (1983).
47. Fourrey et al., *Tetrahedron Lett.*, *24*, 1963—1966 (1983).

**Claims**

1. A method for the preparation of a nucleoside, phosphoramidite intermediate represented by a formula (IV) characterised by the chemical reaction

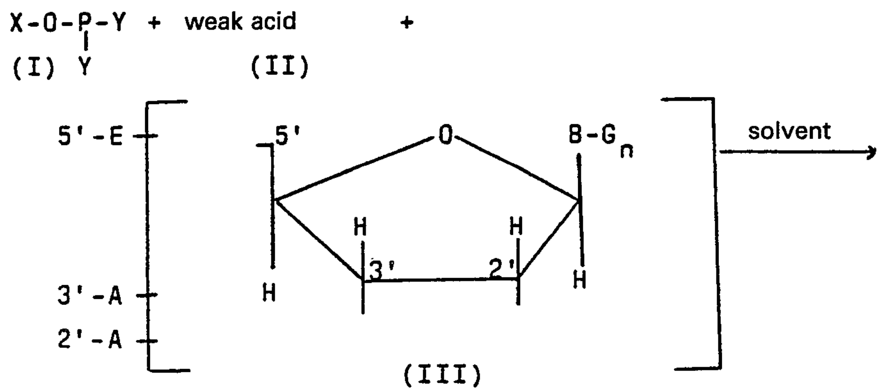

(I) (II)

(III)

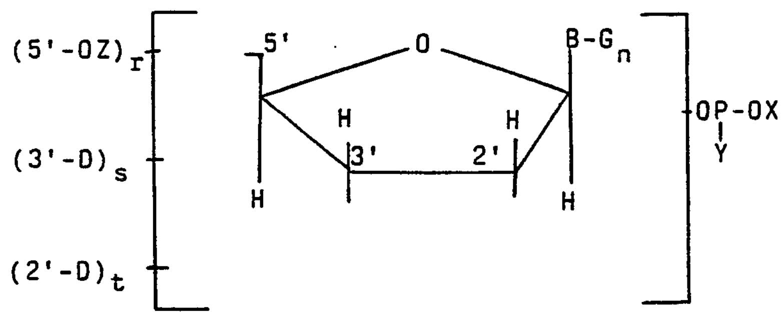

(IV)

wherein:
X is a phosphate protecting group;
each Y is a secondary amino group;
Z is a hydroxy protecting group;
said weak acid is capable of selectively protonating only one amine of said secondary amino groups;
each A is selected from a group consisting of —H, —OH, and —OZ;
B is selected from a group consisting of natural and unnatural purine and pyrimidine bases;
G is a heterocyclic base protecting group;
each D is selected from a group consisting of —H and —OZ;
E is selected from a group consisting of —OH and —OZ, provided that when (a) both As are the same and are —H or —OZ, then E is —OH, and (b) when one A is —OH, then E is OZ;
n is a number from 0—2;
r, s, and t are each 0 or 1, provided that r + s + t = 2;
said solvent is capable of (a) solubilizing I, II, and III and (b) allowing said reaction to proceed; and
III is present in a molar concentration at least equal to the molar concentration of reactable I.

2. The method of claim 1 wherein
III is present in a molar concentration greater than reactable I; and
II is present in a molar concentration at least twice the molar concentration of I.

3. The method of claim 2 wherein II is present in a molar concentration from about 2 to about 5 times the molar concentration of I.

4. The method of claim 3 wherein the molar ratio of III:reactable I is about 1.2:1 and the molar ratio of II:I is about 4:1.

5. The method of claim 1 wherein said weak acid has a pK at about 25°C in $H_2O$ of about 7.5 to about 11.

6. The method of claim 5 wherein said pK of said weak acid is about 8 to about 10.5.

7. The method of claim 6 wherein said pK of said weak acid is about 8 to about 9.

8. The method of claim 7 wherein said pK of said weak acid is about 8.2 to about 8.5.

9. The method of claim 1 wherein each Y is $R_1$—N—$R_2$, each $R_1$ and $R_2$, when taken separately, represents alkyl, haloalkyl, alkenyl, haloalkenyl, aralkyl, haloaralkyl, cycloalkyl, halocycloalkyl, cycloalkylalkyl, halocycloalkylalkyl, cycloalkenylyl, halocycloalkenylyl containing up to 10 carbon atoms, wherein each halogen substitution is at least three carbon atoms removed from the nitrogen atom and each unsaturation is at least 2 carbon atoms from the nitrogen atom; $R_1$ and $R_2$, when taken together, form a chain selected from a group consisting of alkylene, haloalkylene, alkenylene, and haloalkenylene chains containing up to 5 carbon atoms in the principal chain and a total of up to 10 carbon atoms with both terminal valence bonds of said chain being attached to said N atom to which $R_1$ and $R_2$ are attached and wherein each halogen substitution atom is at least three carbon atoms removed from the nitrogen atom and each unsaturation is at least 2 carbon atoms from the nitrogen atom; $R_1$ and $R_2$, when taken together with said N atom to which they are attached, form a saturated nitrogen heterocycle including at least one addition heteroatom selected from a group consisting of N, O, and S; said weak acid has a pK at 25°C in $H_2O$ of about 7.5 to about 11; said solvent is selected from a group consisting of tetramethyl urea, N,N-dimethylacetamide, 1-methyl-2-pyrrolidinone, N,N-dimethylformamide, dioxane, tetrahydrofuran ethyl acetate, chloroform, 1,3-dimethyl-2-imidazolidinone, N,N'-dimethyl-N,N'-propylene urea, homologues and analogues thereof and mixtures thereof; III is present in a molar concentration greater than reactable I; and II is present in a molar concentration at least twice the molar concentration of I.

10. The method of claim 9 wherein each Y is a secondary amino radical of dimethylamine diethylamine, diisopropylamine, dibutylamine, methylpropylamine, methylhexylamine, methylcyclopropylamine, ethylcyclohexylamine, methylbenzylamine, methylcyclohexylmethylamine, butylcyclohexylamine, morpholine, thiomorpholine, pyrolidine, piperidine, or 2,6-dimethylpiperidine; said pK of said acid is about 8 to about 10.5; and II is present in a molar concentration from about 2 to about 5 times the molar concentration of I.

11. The method of claim 10 wherein said pK of said weak acid is about 8 to about 9.

12. The method of claim 11 wherein said pK of said weak acid is about 8.2 to about 8.5

13. The method of claim 12 wherein said weak acid is selected from a group consisting of 1,2,4-triazole; 1,2,3-triazole; 4,5-dichloroimidazole; 4-nitroimidazole; 3-chlorotriazole; benzotriazole; and mixtures thereof.

14. The method of claim 13 wherein said weak acid is selected from said group consisting of 4,5-dichloroimidazole, benzotriazole, and mixtures thereof.

15. The method of claim 6 wherein said weak acid is 4,5-dichloroimidazole; said solvent is selected from a group consisting of tetramethyl urea, 1,3-dimethyl-2-imidazolidinone, 1-methyl-2-pyrrolidinone, and mixtures thereof; the molar ratio of III: reactable I is about 1.2:1 and the molar ratio of II:I is about 4:1.

16. A method for the preparation of a nucleoside phosphoramidite intermediate represented by a formula (IV) characterised by the chemical reaction

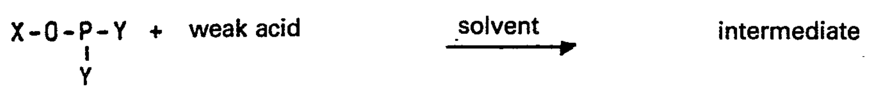


(I) (II)

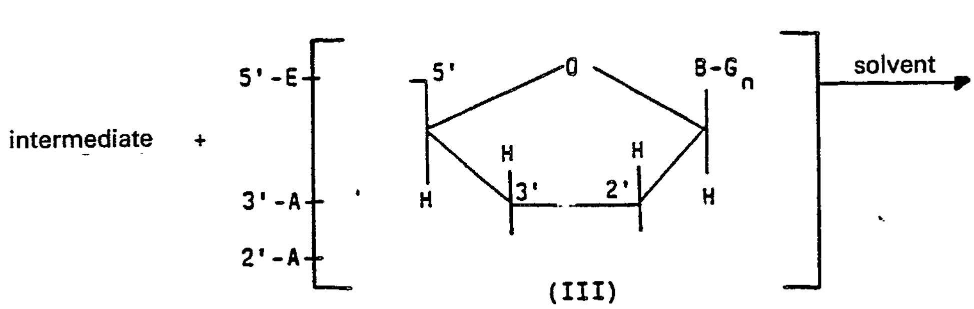


(III)

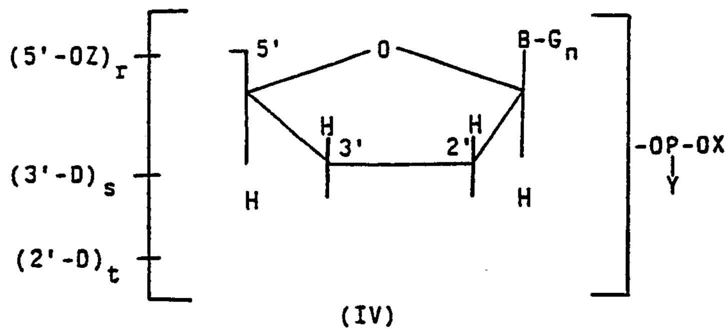

(IV)

wherein:

X is a phosphate protecting group;

each Y is a secondary amino group;

Z is a hydroxy protecting group;

said weak acid is capable of selectively protonating only one amine of said secondary amino groups;

each A is selected from a group consisting of —H, —OH, and —OZ;

B is selected from a group consisting of natural and unnatural purine and pyrimidine bases;

G is a heterocyclic base protecting group;

each D is selected from a group consisting of —H and —OZ;

E is selected from a group consisting of —OH and —OZ, provided that when (a) both As are the same and are —H or —OZ, then E is —OH, and (b) when one A is —OH, then E is OZ;

n is a number from 0—2;

r, s, and t are ech 0 or 1, provided that r + s + t = 2;

said solvent is capable of (a) solubilizing I, II, and III and (b) allowing said reaction to proceed; and III is present in a molar concentration at least equal to the molar concentration of reactable I.

17. The method of claim 16 wherein III is present in a molar concentration greater than reactable I; and II is present in a molar concentration at least twice the molar concentration of I.

18. The method of claim 17 wherein II is present in a molar concentration from about 2 to about 5 times the molar concentration of I.

19. The method of claim 18 wherein the molar ratio of III:reactable I is about 1.2:1 and the molar ratio of II:I is about 4:1.

20. The method of claim 16 wherein said weak acid has a pK at about 25°C in $H_2O$ of about 7.5 to about 11.

21. The method of claim 20 wherein said weak acid has said pK of about 8 to about 10.5.

22. The method of claim 21 wherein said weak acid has said pK of about 8 to about 9.

23. The method of claim 22 wherein said weak acid has said pK of about 8.2 to about 8.5.

24. The method of claim 16 wherein each Y is

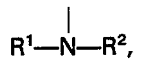

each $R^1$ and $R^2$, when taken separately, represents alkyl, haloalkyl, alkenyl, haloalkenyl, aralkyl, haloaralkyl, cycloalkyl, halocycloalkyl, cycloalkylalkyl, halocycloalkylalkyl, cycloalkenylyl, halocycloalkenylyl containing up to 10 carbon atoms, wherein each halogen substitution is at least three carbon atoms removed from the nitrogen atom and each unsaturation is at least 2 carbon atoms from the nitrogen atom; $R_1$ and $R_2$, when taken together, form a chain selected from a group consisting of alkylene, haloalkylene, alkenylene, and haloalkenylene chains containing up to 5 carbon atoms in the principal chain and a total of up to 10 carbon atoms with both terminal valence bonds of said chain being attached to said N atom to which $R_1$ and $R_2$ are attached and wherein each halogen substitution is at least three carbon atoms removed from the nitrogen atom and each unsaturation is at least 2 carbon atoms from the nitrogen atom; $R_1$ and $R_2$, when taken together with said N atom to which they are attached, form a saturated nitrogen heterocycle including at least one addition heteroatom selected from a group consisting of N, O, and S; said weak acid has a pK at 25°C in $H_2O$ of about 7.5 to about 11; said solvent is selected from a group consisting of tetramethyl urea, N,N-dimethylacetamide, 1-methyl-2-pyrrolidinone, N,N-dimethylformamide, dioxane, tetrahydrofuran ethyl acetate, chloroform, 1,3-dimethyl-2-imidazolidinone, N,N'-dimethyl-N,N'-propylene urea, homologues and analogues thereof and mixtures thereof; III is present in a molar concentration greater than reactable I; and II is present in a molar concentration at least twice the moler concentration of I.

25. The method of claim 24 wherein each Y is a secondary amino radical of dimethylamine diethylamine, diisopropylamine, dibutylamine, methylpropylamine, methylhexylamine, methylcyclopropylamine, ethylcyclohexylamine, methylbenzylamine, methylcyclohexylmethylamine, butylcyclohexylamine, morpholine, thiomorpholine, pyrolidine, piperidine, or 2,6-dimethylpiperidine; said pK of said acid is about 8 to about 10.5; II is present in a molar concentration from about 2 to about 5 times the molar concentration of I.

26. The method of claim 25 wherein said pK of said weak acid is about 8 to about 9.

27. The method of claim 26 wherein said pK of said weak acid is about 8.2 to about 8.5

28. The method of claim 27 wherein said weak acid is selected from a group consisting of 1,2,4-triazole; 1,2,3-triazole; 4,5-dichloroimidazole; 4-nitroimidazole; 3-chlorotriazole; benzotriazole; and mixtures thereof.

29. The method of claim 28 wherein said weak acid is selected from said group consisting of 4,5-dichloroimidazole, benzotriazole, and mixtures thereof.

30. The method of claim 29 wherein said weak acid is 4,5-dichloroimidazole; said solvent is selected from a group consisting of tetramethyl urea, 1,3-dimethyl-2-imidazolidinone, 1-methyl-2-pyrrolidinone, and mixtures thereof; the molar ratio of III: reactable I is about 1.2:1 and the molar ratiof of II:I is about 4:1.

31. A compound having a formula

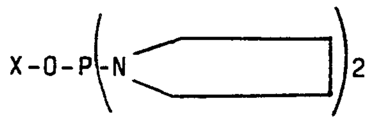

wherein X is methyl.

**Patentansprüche**

1. Verfahren zur Herstellung eines durch eine Forel (IV) wiedergegebenen Nucleosid-Phosphoramidit-Zwischenprodukts, gekennzeichnet durch die chemische Reaktion

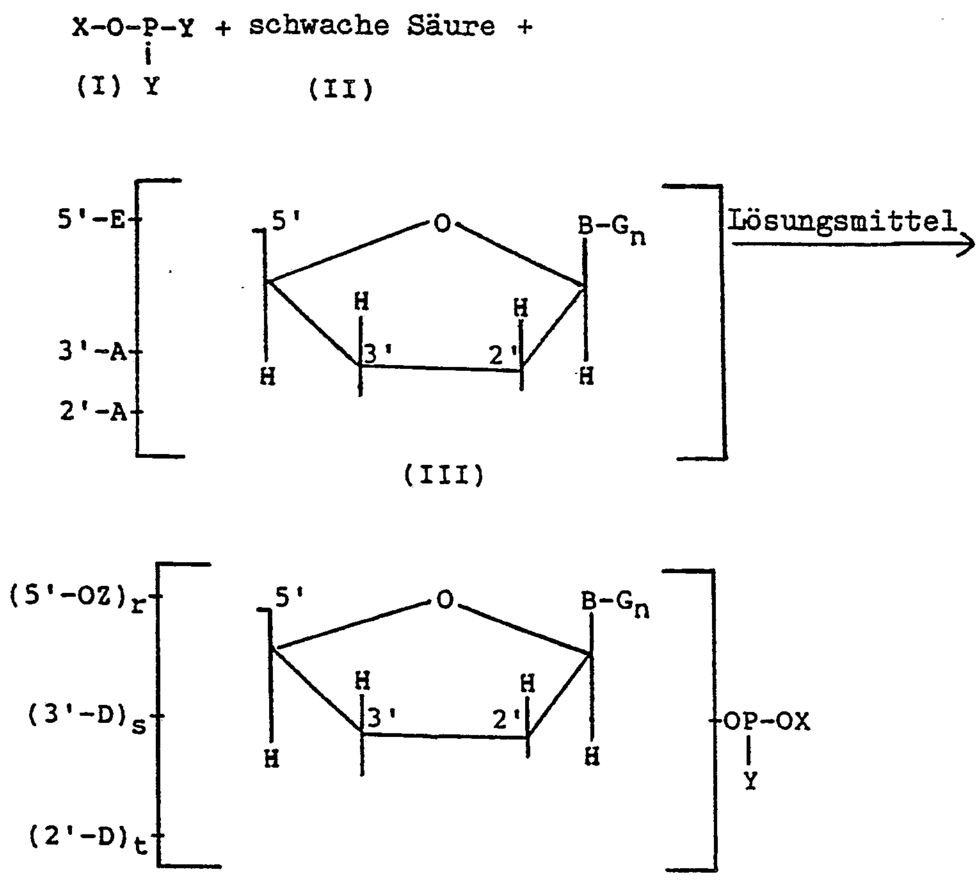


(I) (II) (III) (IV)

wobei:

X eine Phosphat schützende Gruppe ist;

Y jeweils eine sekundäre Aminogruppe ist;

Z eine Hydroxyl schützende Gruppe ist;

die genannte schwache Säure selektiv nur ein Amin der genannten sekundären Amino-Gruppen zu protonieren vermag;

A jeweils aus einer Gruppe gewählt ist, die aus —H, —OH und —OZ besteht;

B aus einer Gruppe gewählt ist, die aus natürlichen und nicht-natürlichen Purin- und Pyrimidin-Basen besteht;

G eine die heterozyklische Base schützende Gruppe ist;

D jeweils aus einer Gruppe gewählt ist, die aus —H und —OZ besteht;

E aus einer Gruppe gewählt ist, die aus —OH und —OZ besteht, mit der Maßgabe, daß dann, wenn (a) die beiden A's gleich sind und —H oder —OZ sind, dann E —OH ist, und (b) wenn ein A —OH ist, dann E OZ ist;

n eine Zahl von 0—2 ist;

r, s und t jeweils 0 oder 1 sind, mit der Maßgabe, daß $r + s + t = 2$;

dass genannte Lösungsmittel (a) I, II und III zu solubilisieren vermag und (b) die genannte Reaktion in ihm ablaufen kann; und

III in einer molaren Konzentration vorliegt, die wenigstens gleich der molaren Konzentration des Reaktanten I ist.

2. Verfahren nach Anspruch 1, wobei III in einer höheren molaren Konzentration als der Reaktant I vorliegt; und II in einer molaren Konzentration vorliegt, die wenigstens das Zweifache der molaren Konzentration von I beträgt.

3. Verfahren nach Anspruch 2, wobei II in einer molaren Konzentration vorliegt, die etwa das 2-fache bis etwa das 5-fache der molaren Konzentration von I beträgt.

4. Verfahren nach Anspruch 3, wobei das molare Verhältnis von III: Reaktant I etwa 1,2:1 und das molare Verhältnis von II:I et 4:1 beträgt.

5. Verfahren nach Anspruch 1, wobei die genannte schwache Säure einen pK-Wert bei etwa 25°C in $H_2O$ von etwa 7,5 bis etwa 11 besitzt.

6. Verfahren nach Anspruch 5, wobei der genannte pK-Wert der schwachen Säure etwa 8 bis etwa 10,5 beträgt.

7. Verfahren nach Anspruch 6, wobei der genannte pK-Wert der genannten schwachen Säure etwa 8 bis etwa 9 beträgt.

8. Verfahren nach Anspruch 7, wobei der genannte pK-Wert der genannten schwachen Säure etwa 8,2 bis etwa 8,5 beträgt.

9. Verfahren nach Anspruch 1, wobei Y jeweils $R_1$—N—$R_2$ ist, $R_1$ und $R_2$ jeweils, für sich gesondert genommen, Alkyl, Haloalkyl, Alkenyl, Haloalkenyl, Aralkyl, Haloaralkyl, Cycloalkyl, Halocycloalkyl, Cycloalkylalkyl, Halocycloalkylalkyl, Cycloalkenylyl, Halocycloalkenylyl mit bis zu 10 Kohlenstoffatomen darstellen, wobei jeweils jede Halogen-Substitution wenigstens 3 Kohlenstoffatome von dem Stickstoffatom entfernt ist und jede Unsättigung wenigstens 2 Kohlenstoffatome von dem Stickstoffatom entfernt ist; $R_1$ und $R_2$ zusammengenommen eine Kette aus der Gruppe der Alkylen-, Haloalkylen-, Alkenylen- und Haloalkenylen-Ketten mit bis zu 5 Kohlenstoffatomen in der Hauptkette und insgesamt bis zu 10 Kohlenstoffatomen bilden, wobei beide endständigen Valenzbindungen der genannten Kette zu dem genannten N-Atom bestehen, an das $R_1$ und $R_2$ gebunden sind, und wobei jeweils jedes Halogen-Substitutionsatom wenigstens 3 Kohlenstoffatome von dem Stickstoffatom entfernt und jeweils jede Unsättigung wenigstens 2 Kohlenstoffatome von dem Stickstoffatom entfernt ist; $R_1$ und $R_2$ zusammengenommen mit dem genannten N-Atom, dem sie zugeordnet sind, einen gesättigten Stickstoff-Heterozyklus bilden, der wenigstens ein zusätzliches Heteroatom aus der Gruppe N, O und S aufweist; die genannte schwache Säure einen pK-Wert bei 25°C in $H_2O$ von etwa 7,5 bis etwa 11 besitzt; das genannte Lösungsmittel aus einer Gruppe gewählt ist, die aus Tetramethylharnstoff, N,N-Dimethylacetamid, 1-Methyl-2-pyrrolidinon, N,N-Dimethylformamid, Dioxan, Tetrahydrofuran, Äthylacetat, Chloroform, 1,3-Dimethyl-2-imidazolidinon, N,N'-Dimethyl-N,N'-propylenharnstoff, deren Homologen und Analogen sowie Gemischen hiervon besteht; III in einer höheren molaren Konzentration als das reaktionsfähige I vorliegt; und II in einer molaren Konzentration vorliegt, die wenigstens das 2-fache der molaren Konzentration von I beträgt.

10. Verfahren nach Anspruch 9, wobei Y jeweils ein sekundäres Amino-Radikal von Dimethylamin, Diäthylamin, Diisopropylamin, Dibutylamin, Methylpropylamin, Methylhexylamin, Methylcyclopropylamin, Äthylcyclohexylamin, Methylbenzylamin, Methylcyclohexylmethylamin, Butylcyclohexylamin, Morpholin, Thiomorpholin, Pyrrolidin, Piperidin oder 2,6-Dimethylpiperidin ist; der genannte pK-Wert der genannten Säure etwa 8 bis etwa 10,5 beträgt; und II in einer molaren Konzentration vorliegt, die das etwa 2-fache bis etwa 5-fache der molaren Konzentration von I ist.

11. Verfahren nach Anspruch 10, wobei der genannte pK-Wert der genannten schwachen Säure etwa 8 bis etwa 9 beträgt.

12. Verfahren nach Anspruch 11, wobei der genannte pK-Wert der genannten schwachen Säure etwa 8,2 bis etwa 8,5 beträgt.

13. Verfahren nach Anspruch 12, wobei die genannte schwache Säure aus einer Gruppe gewählt ist, die aus 1,2,4-Triazol; 1,2,3-Triazol; 4,5-Dichlorimidazol; 4-Nitroimidazol; 3-Chlortriazol; Benzotriazol sowie Gemischen hiervon besteht.

14. Verfahren nach Anspruch 13, wobei die genannte schwache Säure aus einer Gruppe gewählt ist, die aus 4,5-Dichlorimidazol, Benzotriazol und Gemischen hiervon besteht.

15. Verfahren nach Anspruch 6, wobei die genannte schwache Säure 4,5-Dichlorimidazol ist; das genannte Lösungsmittel aus einer Gruppe gewählt ist, die aus Tetramethyl-Harnstoff, 1,3-Dimethyl-2-imidazolidinon, 1-Methyl-2-pyrrolidinon und Gemischen hiervon besteht; das molare Verhältnis von III:reaktionsfähigem I etwa 1,2:1 und das molare Verhältnis von II:I etwa 4:1 beträgt.

16. Verfahren zur Herstellung eines durch eine Formel (IV) wiedergegebenen Nucleosid-Phosphoramidit-Zwischenprodukts, gekennzeichnet durch die chemische Reaktion

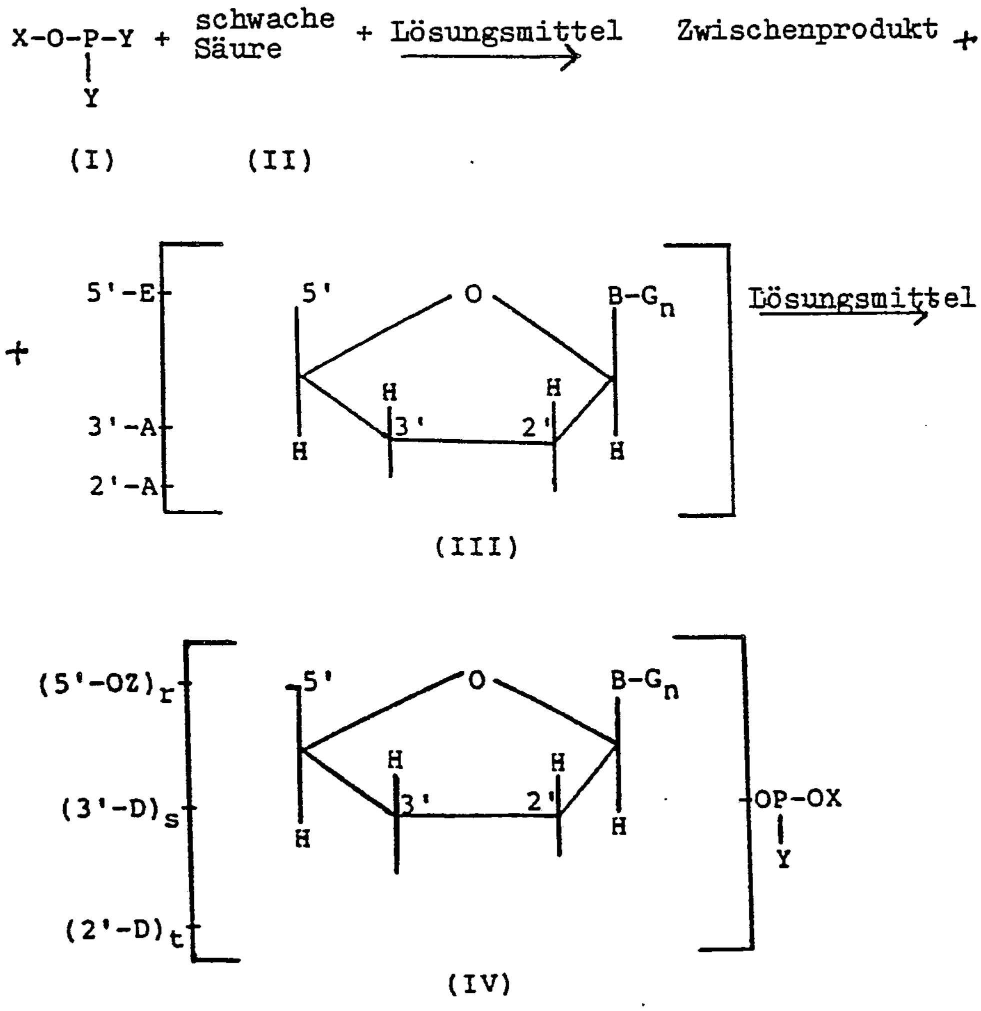


(I) (II) (III) (IV)

wobei:

X eine Phosphat schützende Gruppe ist;

Y jeweils eine sekundäre Aminogruppe ist;

Z eine Hydroxyl schützende Gruppe ist;

die genannte schwache Säure selektiv nur ein Amin der genannten sekundären Amino-Gruppen zu protonieren vermag;

A jeweils aus einer Gruppe gewählt ist, die aus —H, —OH und —OZ besteht;

B aus einer Gruppe gewählt ist, die aus natürlichen und nicht-natürlichen Purin- und Pyrimidin-Basen besteht;

G eine die heterozylklische Base schützende Gruppe ist;

D jeweils aus einer Gruppe gewählt ist, die aus —H und —OZ besteht;

E aus einer Gruppe gewählt ist, die aus —OH und —OZ besteht, mit der Maßgabe, daß dann, wenn (a) die beiden A's gleich sind und —H oder —OZ sind, dann E —OH ist, und (b) wenn ein A —OH ist, dann E OZ ist;

n eine Zahl von 0—2 ist;

r, s und t jeweils 0 oder 1 sind, mit der Maßgabe, daß $r + s + t = 2$;

das genannte Lösungsmittel (a) I, II und III zu solubilisieren vermag und (b) die genannte Reaktion in ihm ablaufen kann; und

III in einer molaren Konzentration vorliegt, die wenigstens gleiche der molaren Konzentration des Reaktanten I ist.

17. Verfahren nach Anspruch 16, wobei III in einer höheren molaren Konzentration als der Reaktant I vorliegt; und II in einer molaren Konzentration vorliegt, die wenigstens das Zweifache der molaren Konzentration von I beträgt.

18. Verfahren nach Anspruch 17, wobei II in einer molaren Konzentration vorliegt, die etwa das 2-fache bis etwa das 5-fache der molaren Konzentration von I beträgt.

19. Verfahren nach Anspruch 18, wobei das molare Verhältnis von III:Reaktant I etwa 1,2:1 und das molare Verhältnis von II:I etwa 4:1 beträgt.

20. Verfahren nach Anspruch 16, wobei die genannte schwache Säure einen pK-Wert bei etwa 25°C in $H_2O$ von etwa 7,5 bis etwa 11 besitzt.

21. Verfahren nach Anspruch 20, wobei der genannte pK-Wert der schwachen Säure etwa 8 bis etwa 10,5 beträgt.

22. Verfahren nach Anspruch 21, wobei der genannte pK-Wert der genannten schwachen Säure etwa 8 bis etwa 9 beträgt.

23. Verfahren nach Anspruch 22, wobei der genannte pK-Wert der genannten schwachen Säure etwa 8,2 bis etwa 8,5 beträgt.

24. Verfahren nach Anspruch 16, wobei Y jeweils $R_1$—N—$R_2$ ist, $R_1$ und $R_2$ jeweils, für sich gesondert genommen, Alkyl, Haloalkyl, Alkenyl, Haloalkenyl, Aralkyl, Haloaralkyl, Cycloalkyl, Halocycloalkyl, Cycloalkylalkyl, Halocycloalkylalkyl, Cycloalkenylyl, Halocycloalkenylyl mit bis zu 10 Kohlenstoffatomen darstellen, wobei jeweils jede Halogen-Substitution wenigstens 3 Kohlenstoffatome von dem Stickstoffatom entfernt ist und jede Unsättigung wenigstens 2 Kohlenstoffatome von dem Stickstoffatom entfernt ist; $R_1$ und $R_2$ zusammengenommen eine Kette aus der Gruppe der Alkylen-, Haloalkylen-, Alkenylen- und Haloalkenylen-Ketten mit bis zu 5 Kohlenstoffatomen in der Hauptkette und insgesamt bis zu 10 Kohlenstoffatomen bilden, wobei beide endständigen Valenzbindungen der genannten Kette zu dem genannten N-Atom bestehen, an das $R_1$ und $R_2$ gebunden sind, und wobei jeweils jedes Halogen-Substitutionsatom wenigstens 3 Kohlenstoffatome von dem Stickstoffatom entfernt und jeweils jede Unsättigung wenigstens 2 Kohlenstoffatome von dem Stickstoffatom entfernt ist; $R_1$ und $R_2$ zusammengenommen mit dem genannten N-Atom, dem sie zugeordnet sind, einen gesättigten Stickstoff-Heterozyklus bilden, der wenigstens ein zusätzliches Heteroatom aus der Gruppe N, O und S aufweist; die genannte schwache Säure einen pK-Wert bei 25°C in $H_2O$ von etwa 7,5 bis etwa 11 besitzt; das genannte Lösungsmittel aus einer Gruppe gewählt ist, die aus Tetramethylharnstoff, N,N-Dimethylacetamid, 1-Methyl-2-pyrrolidinon, N,N-Dimethylformamid, Dioxan, Tetrahydrofuran, Äthylacetat, Chloroform, 1,3-Dimethyl-2-imidazolidinon, N,N'-Dimethyl-N,N'-propylenharnstoff, deren Homologen und Analogen sowie Gemischen hiervon besteht; III in einer höheren molaren Konzentration als das reaktionsfähige I vorliegt; und II in einer molaren Konzentration vorliegt, die wenigstens das 2-fache der molaren Konzentration von I beträgt.

25. Verfahren nach Anspruch 24, wobei Y jeweils ein sekundäres Amino-Radikal von Dimethylamin, Diäthylamin, Diisopropylamin, Dibutylamin, Methylpropylamin, Methylhexylamin, Methylcyclopropylamin, Äthylcyclohexylamin, Methylbenzylamin, Methylcyclohexylmethylamin, Butylcyclohexylamin, Morpholin, Thiomorpholin, Pyrrolidin, Piperidin oder 2,6-Dimethylpiperidin ist; der genannte pK-Wert der genannten Säure etwa 8 bis etwa 10,5 beträgt; und II in einer molaren Konzentration vorliegt, die das etwa 2-fache bis etwa 5-fache der molaren Konzentration von I ist.

26. Verfahren nach Anspruch 25, wobei der genannte pK-Wert der genannten schwachen Säure etwa 8 bis etwa 9 beträgt.

27. Verfahren nach Anspruch 26, wobei der genannte pK-Wert der genannten schwachen Säure etwa 8,2 bis etwa 8,5 beträgt.

28. Verfahren nach Anspruch 27, wobei die genannte schwache Säure aus einer Gruppe gewählt ist, die aus 1,2,4-Triazol; 1,2,3-Triazol; 4,5-Dichlorimidazol; 4-Nitroimidazol; 3-Chlortriazol; Benzotriazol sowie Gemischen hiervon besteht.

29. Verfahren nach Anspruch 28, wobei die genannte schwasche Säure aus einer Gruppe gewählt ist, die aus 4,5-Dichlorimidazol, Benzotriazol und Gemischen hiervon besteht.

30. Verfahren nach Anspruch 29, wobei die genannte schwache Säure 4,5-Dichlorimidazol ist; das genannte Lösungsmittel aus einer Gruppe gewählt ist, die aus Tetramethyl-Harnstoff, 1,3-Dimethyl-2-imidazolidinon, 1-Methyl-2-pyrrolidinon und Gemischen hiervon besteht; das molare Verhältnis von III:reaktionsfähigem I etwa 1,2:1 und das molare Verhältnis von II:I etwa 4:1 beträgt.

31. Verbindung entsprechend der Formel

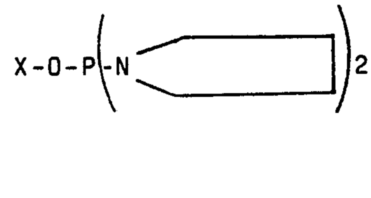

wobei X Methyl ist.

## Revendications

1. Méthode pour la préparation d'un intermédiaire de nucléoside phosphoramidite représenté par une formule (IV) caractérisée par la réaction chimique

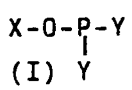
+ acide faible +

(I) (II)

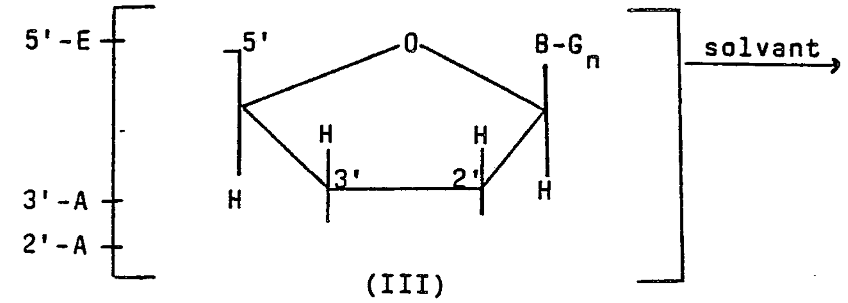

(III)

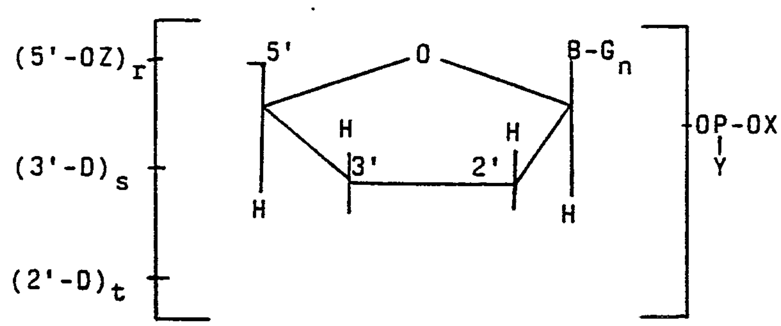

(IV)

où

X est un groupe protecteur de phosphate; chaque Y est un groupe amino secondaire;

Z est un groupe hydroxy protecteur;

ledit acide faible est capable de protoner sélectivement une seule amine dudit groupe amino secondaire;

chaque A est choisi dans un groupe consistant en —H, —OH et —OZ;

B est choisi dans un groupe consistant en bases de purine et de pyrimidine naturelles et non naturelles;

G est un groupe protecteur de base hétérocyclique;

chaque D est choisi dans un groupe consistant en —H et —OZ;

E est choisi dans un groupe consistant en —OH et —OZ, à condition que quand (a) les deux A sont identiques et sont —H ou —OZ, alors E soit —OH et (b) quand un A est —OH, alors E soit OZ;

n est un nombre de 0—2;

r, s et t sont chacun 0 ou 1 à condition que $r + s + t = 2$;

ledit solvant est capable de (a) solubiliser I, II et III et (b) permettre à la réaction de se passer; et

III est présent à une concentration molaire au moins égale à la concentration molaire de I pouvant réagir.

2. Méthode selon la revendication 1 où III est présent à une concentration molaire plus importante que I pouvant réagir; et II est présent à une concentration molaire au moins le double de la concentration molaire de I.

3. Méthode selon la revendication où II est présent à une concentration molaire d'environ 2 à environ 5 fois la concentration molaire de I.

4. Méthode selon la revendication 3 où le rapport molaire de III:I pouvant réagir est d'environ 1,2:1 et le rapport molaire de II:I est d'environ 4:1.

5. Méthode selon la revendication 1 où ledit acide faible a un pK à environ 25°C dans $H_2O$ d'environ 7,5 à environ 11.

6. Méthode selon la revendication 5 où ledit pK dudit acide faible est d'environ 8 à environ 10,5.

7. Méthode selon la revendication 6 où ledit pK dudit acide faible est d'environ 8 à environ 9.

8. Méthode selon la revendication 7 où ledit pK dudit acide faible est d'environ 8,2 à environ 8,5.

9. Méthode selon la revendication 1 où chaque de Y est $R_1$—N—$R_2$, chacun de $R_1$ et $R_2$, lorsqu'ils sont pris séparément, représente alkyle, haloalkyle, alkényle, haloalkényle, aralkyle, haloaralkyle, cycloalkyle, halocycloalkyle, cycloalkylalkyle, halocycloalkylalkyle, cycloalkénylyle, halocycloalkénylyle contenant jusqu'à 10 atomes de carbone, où chaque substitution d'halogène est à au moins trois atomes de carbone de l'atome d'azote et chaque insaturation est à au moins deux atomes de carbone de l'atome d'azote; $R_1$ et $R_2$, pris ensemble, forment une chaîne choisie dans un groupe consistant en chaînes d'alkylène, haloalkylène, alkénylène et haloalkénylène contenant jusqu'à 5 atomes de carbone dans la chaîne principale et un total pouvant atteindre 10 atomes de carbone avec les deux liaisons de valence terminale de ladite chaîne qui sont attachées audit atome de N auquel $R_1$ et $R_2$ sont attachés et où chaque atome de substitution d'halogène est à au moins trois atomes de carbone de l'atome d'azote et chaque insaturation

est à au moins deux atomes de carbone de l'atome d'azote; $R_1$ et $R_2$, lorsqu'ils sont pris ensemble avec ledit atome de N auquel ils sont attachés, forment un hétérocycle saturé d'azote comprenant au moins un hétéroatome d'addition choisi dans un groupe consistant en N, O et S; ledit acide faible a un pK à 25°C dans $H_2O$ d'environ 7,5 à environ 11; ledit solvant est choisi dans un groupe consistant en tétraméthyl urée, N,N-diméthylacétamide, 1-méthyl-2-pyrrolidinone, N,N-diméthylformamide, dioxane, tétrahydrofuranne, acétate d'éthyle, chloroforme, 1,3-diméthyl-2-imidazolidinone, N,N'-diméthyl-N,N'-propylène urée, leurs homologues et analogues, et leurs mélanges; III est présent à une concentration molaire plus importante que I pouvant réagir; et II est présent à une concentration molaire qui est au moins le double de la concentration molaire de I.

10. Méthode selon la revendication 9 où chaque Y est un radical amino secondaire de diméthylamine, diéthylamine, diisopropylamine, dibutylamine, méthylpropylamine, méthylhexylamine, méthylcyclopropylamine, éthylcyclohexylamine, méthylbenzylamine, méthylcyclohexylméthylamine, butylcyclohexylamine, morpholine, thiomorpholine, pyrrolidine, pipéridine ou 2,6-diméthylpipéridine; ledit pK dudit acide est d'environ 8 à environ 10,5; et II est présent à une concentration molaire d'environ 2 à environ 5 fois la concentration molaire de I.

11. Méthode de la revendication 10 où ledit pK dudit acide faible est d'environ 8 à environ 9.

12. Méthode de la revendication 11 où ledit pK dudit acide faible est d'environ 8,2 à environ 8,5.

13. Méthode de la revendication 12 où ledit acide faible est choisi dans un groupe consistant en 1,2,4-triazole; 1,2,3-triazole; 4,5-dichloroimidazole; 4-nitroimidazole; 3-chlorotriazole; benzotriazole; et leurs mélanges.

14. Méthode selon la revendication 13 où ledit acide faible est choisi dans le groupe consistant en 4,5-dichloroimidazole, benzotriazole et leurs mélanges.

15. Méthode de la revendication 6 où ledit acide faible est 4,5-dichloroimidazole; ledit solvant est choisi dans un groupe consistant en tétraméthyl urée, 1,3-diméthyl-2-imidazolidinone, 1-méthyl-2-pyrrolidinone et leurs mélanges; le rapport molaire de III:I pouvant réagir est d'environ 1,2:1 et le rapport molaire de II:I est d'environ 4:1.

16. Méthode pour la préparation d'un intermédiaire de nucléoside phosphoramidite représenté par une formule (IV) caractérisée par la réaction chimique

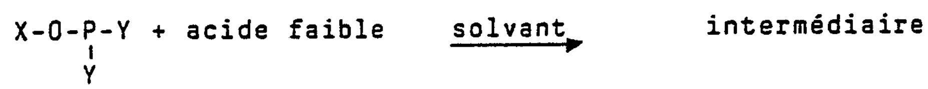


(I) (II)

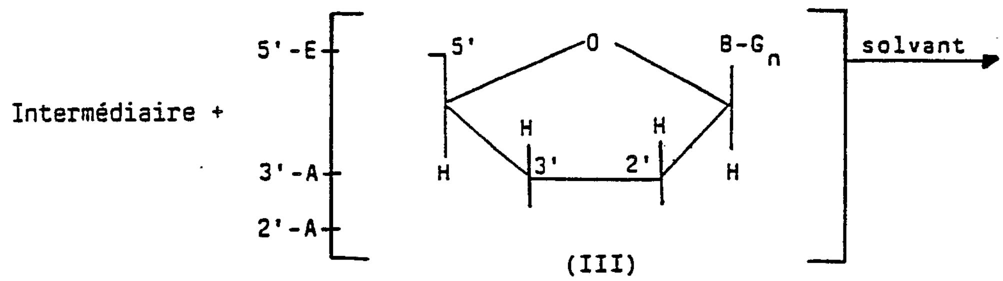


(III)

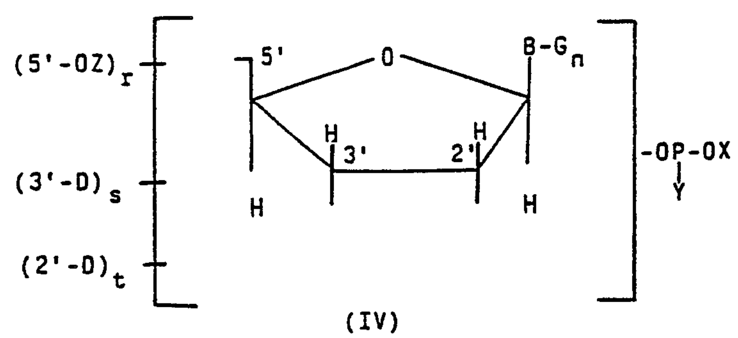

(IV)

où

X est un groupe protecteur de phosphate; chaque Y est un groupe amino secondaire;

Z est un groupe hydroxy protecteur;

ledit acide faible est capable de protoner sélectivement une seule amine dudit groupe amino secondaire;

chaque A est choisi dans un groupe consistant en —H, —OH et —OZ;

B est choisi dans un groupe consistant en bases de purine et de pyrimidine naturelles et non naturelles;

G est un groupe protecteur de base hétérocyclique;

chaque D est choisi dans un groupe consistant en —H et —OZ;

E est choisi dans un groupe consistant en —OH et —OZ, à condition que quand (a) les deux A sont identiques et sont —H ou —OZ, alors E soit —OH et (b) quand un A est —OH, alors E soit —OZ;

n est un nombre de 0—2;

r, s et t sont chacun 0 ou 1 à condition que r + s + t = 2;

ledit solvant est capable de (a) solubiliser I, II et III et (b) permettre à la réaction de se passer; et

III est présent à une concentration molaire au moins égale à la concentration molaire de I pouvant réagir.

17. Méthode de la revendication 16 où III est présent à une concentration molaire plus importante que I pouvant réagir; et II est présent à une concentration molaire au moins le double de la concentration molaire de I.

18. Méthode selon la revendication 17 où II est présent à une concentration molaire d'environ 2 à environ 5 fois la concentration molaire de I.

19. Méthode selon la revendication 18 où le rapport molaire de III:I pouvant réagir est d'environ 1,2:1 et le rapport molaire de II:I est d'environ 4:1.

20. Méthode selon la revendication 16 où ledit acide faible a un pK à environ 25°C dans $H_2O$ d'environ 7,5 à environ 11.

21. Méthode selon la revendication 20 où ledit pK dudit acide faible est d'environ 8 à environ 10,5.

22. Méthode selon la revendication 21 où ledit pK dudit acide faible est d'environ 8 à environ 9.

23. Méthode selon la revendication 22 où ledit pK dudit acide faible est d'environ 8,2 à environ 8,5.

24. Méthode selon la revendication 16 où chaqun de Y est

|
$R_1$—N—$R_2$,

chacun de $R_1$ et $R_2$, lorsqu'ils sont pris séparément, représente alkyle, haloalkyle, alkényle, haloalkényle, aralkyle, haloaralkyle, cycloalkyle, halocycloalkyle, cycloalkylalkyle, halocycloalkylalkyle, cycloalkénylyle, halocycloalkénylyle contenant jusqu'à 10 atomes de carbone, où chaque substitution d'halogène est à au moins trois atomes de carbone de l'atome d'azote et chaque insaturation est à au moins deux atomes de carbone de l'atome d'azote; $R_1$ et $R_2$, pris ensemble, forment une chaîne choisie dans un groupe consistant en chaînes d'alkylène, haloalkylène, alkénylène et haloalkénylène contenant jusqu'à 5 atomes de carbone dans la chaîne principale et un total pouvant atteindre 10 atomes de carbone avec les deux liaisons de valence terminale de ladite chaîne qui sont attachées audit atome de N auquel $R_1$ et $R_2$ sont attachés et où chaque atome de substitution d'halogène est à au moins trois atomes de carbone de l'atome d'azote et chaque insaturation est à au moins deux atomes de carbone de l'atome d'azote; $R_1$ et $R_2$, lorsqu'ils sont pris ensemble avec ledit atome de N auquel ils sont attachés, forment un hétérocycle saturé d'azote comprenant au moins un hétéroatome d'addition choisi dans un groupe consistant en N, O et S; ledit acide faible a un pK à 25°C dans $H_2O$ d'environ 7,5 à environ 11; ledit solvant est choisi dans un groupe consistant en tétraméthyl urée, N,N-diméthylacétamide, 1-méthyl-2-pyrrolidinone, N,N-diméthylformamide, dioxane, tétrahydrofuranne, acétate d'éthyle, chloroforme, 1,3-diméthyl-2-imidazolidinone, N,N'-diméthyl-N,N'-propylène urée, leurs homologues et analogues, et leurs mélanges; III est présent à une concentration molaire plus importante que I pouvant réagir; et II est présent à une concentration molaire qui est au moins le double de la concentration molaire de I.

25. Méthode selon la revendication 24 où chaque Y est un radical amino secondaire de diméthylamine, diéthylamine, diisopropylamine, dibutylamine, méthylpropylamine, méthylhexylamine, méthylcyclopropylamine, éthylcyclohexylamine, méthylbenzylamine, méthylcyclohexylméthylamine, butylcyclohexylamine, morpholine, thiomorpholine, pyrrolidine, pipéridine ou 2,6-diméthylpipéridine; ledit pK dudit acide est d'environ 8 à environ 10,5; et II est présent à une concentration molaire d'environ 2 à environ 5 fois la concentration molaire de I.

26. Méthode selon la revendication 25 où ledit pK dudit acide faible est d'environ 8 à environ 9.

27. Méthode selon la revendication 26 où ledit pK dudit acide faible est d'environ 8,2 à environ 8,5.

28. Méthode selon la revendication 27 où ledit acide faible est choisi dans un groupe consistant en 1,2,4-triazole; 1,2,3-triazole; 4,5-dichloroimidazole; 4-nitroimidazole; 3-chlorotriazole; benzotriazole; et leurs mélanges.

29. Méthode selon la revendication 28 où ledit acide faible est choisi dans un groupe consistant en 4,5-dichloroimidazole, benzotriazole et leurs mélanges.

30. Méthode selon la revendication 28 où ledit acide faible est 4,5-dichloroimidazole; ledit solvant est choisi dans le groupe consistant en tétraméthyl urée, 1,3-diméthyl-2-imidazolidinone, 1-méthyl-2-pyrrolidinone et leurs mélanges; le rapport molaire de III:I pouvant réagir est d'environ 1,2:1 et le rapport molaire de II:I est d'environ 4:1.

31. Composé ayant pour formule

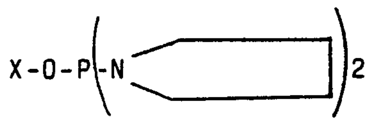

où X est méthyle.